# EUROPEAN PATENT APPLICATION

(11) **EP 3 045 525 A1**
(43) Date of publication of application: **20.07.2016**
(21) Application number: 15199863.0
(22) Date of filing: 14.12.2015
(51) Int. Cl.: C12M 1/107, C12M 1/00

(54) **PLANT FOR THE PRODUCTION OF GAS**

(30) Priority: 12.12.2014 IT UD20140188
(71) Applicant: Poopy3energy S.r.l., 34148 Trieste (IT)
(72) Inventor: Dionis, Erica, 34148 Trieste (IT)
(74) Representative: Petraz, Gilberto Luigi

(57) **Abstract**

Plant for the production of gas, by means of digestion of an organic material, comprising a first container (11) configured to contain said organic material and in which at least part of the digestive process occurs. The plant comprises at least a second container (12) and a first transfer device (35) connected between the first container (11) and the second container (12) and configured to transfer the organic material from the first container (11) to the second container (12). To at least one of either the first container (11) or the second container (12) a respective first recirculation member (24, 43) is connected, configured to generate a recirculation of the organic material in the respective container.

## Description

### FIELD OF THE INVENTION

The present invention concerns a plant for the production of gas, such as a digester, configured to perform a process to digest organic material, for example but not only deriving from zootechnical residues such as animal excreta and/or discards of agricultural production, to generate biogas containing at least methane.

In particular, the plant according to the present invention is able to perform a substantially continuous anaerobic digestion process of the material conferred therein.

The present invention also concerns a method for the production of gas deriving from organic residues.

### BACKGROUND OF THE INVENTION

Processes of anaerobic digestion of organic materials are known, which comprise four main stages in each of which the organic mass is progressively dissociated into simpler and simpler compounds.

In particular, the anaerobic digestion process comprises:
- a first stage of hydrolysis, in which the organic molecules are split into simpler compounds such as monosaccharides, amino acids and fatty acids;
- a second stage, called acidogenesis, where a further splitting occurs into even simpler molecules, such as volatile fatty acids (for example acetic, propionic, butyric and valeric acid), with the production of ammonia, carbon dioxide and hydrogen sulfide as sub-products;
- a third stage, called acetogenesis, where the simple molecules produced in the previous stage are further digested producing carbon dioxide, hydrogen, and mainly acetic acid;
- fourth and last stage, called methanogenesis, during which methane, carbon dioxide and water are produced.

Plants are also known that exploit the anaerobic digestion process described above to produce gas starting from an organic material.
In particular these known plants are the monophase type, i.e., all four stages of hydrolysis, acidogenesis, acetogenesis and methanogenesis take place in the same plant.

Monophase plants comprise a single container, also called single-stage digester, in which the whole anaerobic digestion process of the material contained therein takes place.

In monophase plants there may also be optionally present a second container, also called post-fermenter, which does not have a digester function proper, but contains all the material not completely digested by the first container which is by now exhausted, to be subsequently discharged.

The presence of a single container is disadvantageous because the bacterial species involved in the different stages of the anaerobic digestion process are multiple, they coexist in a single space, and create contemporaneity and overlapping of the bacterial populations belonging to the various phases.

Consequently, known plants do not allow to optimize the growth of one bacterial species rather than another, and therefore the latter are not able to perform a complete digestion of all the materials, in particular of foods that are rich in vegetable fibers. This gives a process that is not very efficient.

It is known for example that methanogen bacteria, which participate in the last anaerobic stage and which give efficiency and reliability to the anaerobic process, have a very slow growth compared with the other bacterial loads, and are commonly considered those most sensitive to toxicity. An environment that is unfavorable to them can therefore generate a drastic decline in the efficiency of the process.

Furthermore, in these monophase plants, the bacterial load contained in the liquid phase arriving from the container is discharged during the process, excluding the possibility of being cultivated.

In known plants, from the biogas obtained at the end of the process only 50-55% of its content in volume is methane, and hence usable as fuel.

Moreover, in known plants the time the organic mass remains in the UASB container is particularly long, it can be as much as sixty days or more, and varies depending on the content of liquid phase and solid phase present in the organic mass at entrance.

Furthermore, as a consequence of this and of the fact that the digestion process is monophase, known plants based on the single stage technology are particularly large, to contain a large quantity of material to be treated.

One purpose of the present invention is to obtain a plant for the production of gas which is highly efficient and reliable.

Another purpose of the present invention is to obtain a plant for the production of gas that allows to reduce the times of the anaerobic digestion process, while still ensuring great efficiency and reliability.

Another purpose of the present invention is to obtain a plant for the production of gas which, given the same efficiency, is more limited in size compared with state-of-the-art plants.

Another purpose of the present invention is to obtain a plant for the production of gas which is economical.

Another purpose of the present invention is to obtain a plant that is compact and transportable for the production of gas which is simple and allows rapid installation.

Another purpose of the present invention is to obtain a plant for the production of gas that is extremely compact and that allows it to be transported to different installation sites.

Another purpose of the present invention is to perfect a method for the production of gas deriving from organic residues that is efficient and reliable.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, a plant for the production of gas, by means of digestion of an organic material, comprises a first container configured to contain the organic material and in which at least part of the digestive process occurs.

In accordance with one aspect of the present invention, the plant comprises at least a second container and a first transfer device connected between the first container and the second container and configured to transfer the organic material from the first container to the second container. In this way it is possible to promote, in each of the two containers, the growth and efficiency of specific bacterial loads that intervene in the digestion and methanogenesis process, preventing overlapping. This allows to increase the efficiency of the gas production process. Moreover, the transfer device allows to have a continuous process and prevents the loss of bacterial load created during the anaerobic digestion process inside each container.

According to another aspect of the present invention a respective first recirculation member, configured to generate a recirculation of the organic material in the respective container, is connected to at least one of either the first container or the second container. This solution allows to increase the efficiency and the development of the bacterial loads in the container, preventing the organic material present in one of the containers from stratifying and not allowing the development of the various reactions needed for the production of gas.

The present invention also concerns a method for the production of gas which provides to introduce and temporarily keep the organic material in a first container in order to start the digestive process.

In accordance with some implementations of the invention the method provides at least to transfer the organic material from the first container into a second container, and to keep the organic material in the second container to allow the continuation of the digestive process.

The method also provides that in at least one of either the first container or the second container a recirculation of the material contained therein is generated.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a schematic representation of the plant for the production of gas in accordance with a possible embodiment;
- figs. 2, 3 and 4 are representations in cross section of components of the plant in accordance with embodiments of the present invention.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

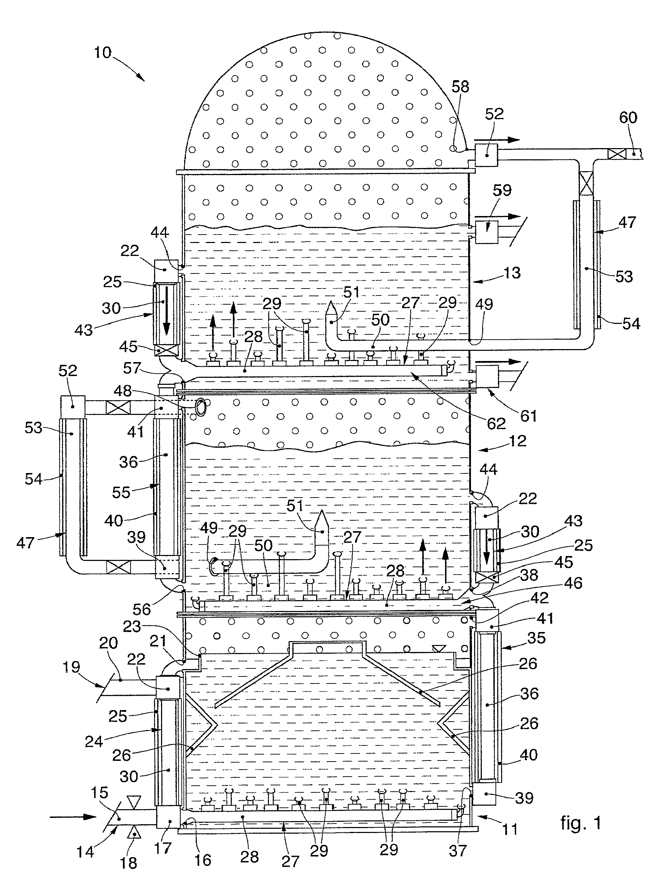

According to the present description, a plant for the production of gas is indicated in its entirety in fig. 1 by the reference number 10, and is configured to treat an organic material, for example of zootechnical origin to produce biogas.

According to the embodiment in fig. 1, the plant for the production of gas 10 comprises at least two containers, in this case three containers respectively a first container 11, a second container 12 and a third container 13, each of which is configured to contain, as described hereafter, the organic material to be processed. In each of the containers successive digestion stages of the organic material inserted take place, by means of a controlled growth of the bacterial load that is generated.

The first container 11, second container 12 and third container 13 are substantially closed over themselves to allow both to contain the fractions of solid/fluid material, and also the gaseous fraction generated.

The first container 11, second container 12 and third container 13, in possible embodiments, can be installed one on top of the other to allow to contain their overall size.

The first container 11, second container 12 and third container 13 can have a containment volume which, merely by way of example, is comprised between 30 m³ and 90 m³.

A feed device 14 is connected to the first container 11, configured to feed the organic material to be processed into the first container 11.

The first container 11, in this case the UASB type, is filled with organic material to a determinate level and an upper part thereof is left free to allow to contain the gas generated during the digestive process in the first container 1 l.

The feed device 14 can be connected to the first container 11 near the bottom wall of the latter. In this way, during the introduction of the organic material into the first container 11, a turbulence is generated in the organic material already present, preventing it from stratifying.

The feed device 14 can comprise, for example, a feed pipe 15 connected to the first container 11, and a first pump 17 configured to introduce into the first container 11 the organic material to be processed.

The feed pipe 15 can be connected in correspondence with an introduction aperture 16 made for example in a perimeter wall of the first container 11, near its bottom wall.

According to a possible solution of the present invention, the feed device 14 can comprise distribution elements 27, located inside the first container 11, in this case in correspondence with its bottom wall, and suitable to distribute uniformly the organic material fed.

The distribution elements 27 are in this case uniformly distributed in the surface development of the cross section of the first container 11.

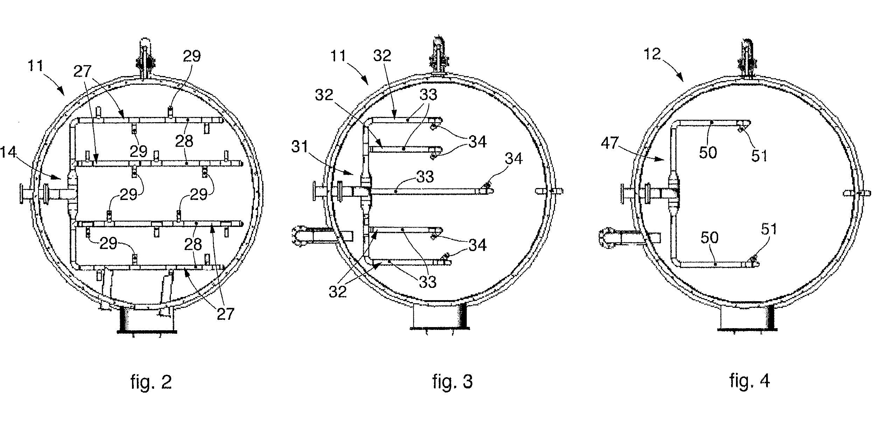

According to the solution in fig. 2, the distribution elements 27 comprise a plurality of tubes 28 connected in a branched manner and each provided with a plurality of distribution nozzles 29 through which the organic material is distributed.

The distribution nozzles 29 can be disposed at different heights from each other and with respect to the tubes 28, so as to obtain a differentiated stirring of the organic material at different heights.

According to possible embodiments, treatment devices 18 (fig. 1) can be associated with the feed device 14 to treat the organic material introduced.

According to possible embodiments, the treatment devices 18 can be chosen from a group comprising a shredder, a crusher, a stirrer, a heater, a homogenizer, or a possible combination thereof.

According to the embodiment in fig. 1, the treatment devices 18 comprise an ultrasound generation member, configured to induce in the organic material ultrasounds suitable to heat the material introduced and to homogenize it.

The first container 11 also comprises a discharge device 19, positioned in an upper part of the first container 11, i.e. distanced from its bottom wall, and configured to discharge at least the liquid part of the organic material introduced into the first container 11.

In fact, the first container 11 can allow to separate the solid part and the liquid part of the material in the first container 11. The solid part and the liquid part are respectively stratified toward the bottom wall and the upper part of the first container 11.

The discharge device 19 can comprise a discharge pipe 20 connected near a discharge aperture 21 provided in an upper part of the wall of the first container 11, and a pumping member 22 connected to the discharge pipe 20 and suitable to suck up at least the liquid part of the material introduced.

According to a possible embodiment, the discharge device 19 can also comprise an overflow element 23, associated with the wall of the first container 11, connected to the discharge aperture 21 and through which at least the liquid part of the material present in the first container 11 is discharged when a predefined maximum containing level is reached.

According to a possible embodiment of the present invention, the plant 10 comprises a first recirculation member 24 interposed between the introduction aperture 16 and the discharge aperture 21, and is configured to allow the material to recirculate in the first container 11.

The first recirculation member 24 can comprise a transfer pipe 30, connected between the introduction aperture 16 and the discharge aperture 21, and a pump, in this case the first pump 17, which allows to reintroduce into the first container 11 the recirculated material. The recirculation action of the organic material can also be obtained either by the action of the pumping member 22, for example by the action of a determinate deflection member, or by a distribution device.

According to a possible solution, the first recirculation member 24 can be directly connected to the feed device 14 to reintroduce at least part of the recirculated material.

According to a variant, the first recirculation member 24 can be connected, instead of to the feed device 14, to a specific stirring device 31 located in the first container 11.

The stirring device 31 is configured to introduce into the first container 11 the recirculated organic material with a desired degree of turbulence and stirring. This prevents the organic material in the first container 11 from stratifying, and optimizes the growth of the bacterial load.

The first pump 17 can be selectively connected to the feed device 14 and to the stirring device 31 by interception or distribution valves, commanded and activated according to the specific function to be activated.

The stirring device 31, according to the embodiment in fig. 3, can comprise a plurality of delivery elements 32 located inside the first container 11, in this case in correspondence with its bottom wall.

The delivery elements 32 can comprise a plurality of tubes 33, connected branch-wise and each provided with one or more delivery nozzles 34 through which the organic material is delivered at a predefined speed and pressure to ensure a stirring action on the organic material contained in the first container 11.

The pumps that feed the feed device have a capacity of 20 m³/hour, while the pumps that feed the stirring system have a capacity of 30 m³/hour.

According to a possible variant, the first recirculation member 24 can be associated with heating devices 25 suitable to heat the liquid material that is recirculated to a minimum temperature of at least 38°C, and is then taken, in the second container 12 and the third container 13, to a temperature, merely by way of example, comprised between 50°C and 52°C.

Possible solutions of the present invention provide that the heating device 25 is chosen from a group comprising at least one of either a heat exchanger, a burner, a resistance, magnetic induction exciters or ultrasound exciters.

According to a possible variant, not shown in the drawings, the heating device 25 can be installed in the first container 11.

According to some embodiments of the present invention, the first container 11 can be provided inside with deflection elements 26 suitable to generate in the organic material deflections in the path that the stream of gas has to follow to move to the surface. In particular the deflection elements 26 obtain a UASB reactor.

In this way it is possible to increase the time the gas which is generated in the liquid organic material remains inside, promoting an optimized growth of the bacterial load in the first container 11.

According to the solution shown in fig. 1, the deflection elements 26 comprise a bell-shaped element installed in a central position of the first container 11 and facing with its concave part toward the bottom wall, and one or more deflection sectors, for example ring-shaped and installed on the periphery of the internal compartment of the first container 11. Between the bell and the deflection sectors at least a passage gap is defined through which the gas is obliged to pass to reach the free surface.

According to the solution shown in fig. 1, a first transfer device 35 is connected to the first container 11 and the second container 12, and is configured to allow the transfer of the organic material from the first container 11 to the second container 12.

According to the solution shown in fig. 1, the first transfer device 35 comprises a tube 36 connected between the first container 11 and the second container 12 to allow the fluidic connection of the two.

The tube 36 can be connected to the first container 11 in correspondence with a pickup aperture 37 and to the second container 12 in correspondence with an introduction aperture 38.

The pickup aperture 37 can be disposed near the bottom wall of the first container 11 to allow to pick up the organic material contained in the latter.

The first transfer device 35 can also comprise a pickup member 39 configured to suck up the organic material from the first container 11 and transfer it through the tube 36 into the second container 12.

According to a possible embodiment of the present invention, a heating device 40 can be associated with the first transfer device 35, substantially analogous to the heating device 25 and configured to heat the organic material transferred.

According to a possible solution, the organic material is heated by the first heating device 25 to a temperature comprised between 50°C and 55°C.

According to a possible variant, not shown in the drawings, the heating device 40 can be installed directly in the first container 11, and/or in the second container 12.

According to a possible solution, a suction member 41 can also be connected to the first container 11, configured to transfer the gas generated in the first container 11 to the second container 12.

The suction member 41 can be connected to a suction aperture 42 made in the first container 11, in this case in an upper part of it where the gas generated stratifies.

According to possible embodiments of the invention, the suction member 41 can be connected to the pickup member 39 to perform a coordinated transfer of both the fluid organic material and also the gas from the first container 11 to the second container 12.

According to a variant, the transfer of the fluid organic material and the transfer of the gas can take place at different moments in time, for example by activating/deactivating distribution or interception valves.

According to this embodiment, the suction member 41 and the pickup member 39 can also be connected to a possible homogenization member, not shown in the drawings, suitable to homogenize the gas with the organic material before it is introduced into the second container 12.

The second container 12 is in turn provided with a feed device 46 configured to feed the organic material and possibly the gas from the first container 11 to the second container 12.

The feed device 46 of the second container 12, like the feed device 14 of the first container 11, can comprise distribution elements 27 substantially analogous to those described above.

A first recirculation member 43 can also be connected to the second container 12, configured to recirculate the organic material, in this case its solid/fluid component contained in the second container 12.

The first recirculation member 43 of the organic material is fundamental to allow the cultivation of the specific bacteria for the execution of the methanogenesis stage so that the production of methane can be maximized.

The first recirculation member 43 is connected in correspondence with a first recirculation aperture 44 and a second recirculation aperture, which in this case corresponds to the introduction aperture 38, made in the second container 12.

The first recirculation aperture 44 is made in an intermediate position along the extension in height of the second container 12, and in correspondence with which, during use, the level of the solid/fluid organic material is positioned.

The first recirculation member 43, like the first recirculation member 24, can comprise the tube 30 and the pumping member 22 as described above.

Heating devices 25 can also be associated with the tube 30, as described above with reference to the first container 11.

An interception valve 45 can be associated with the tube 30, to intercept the flow of material passing through it, which can be closed to allow the organic material to be introduced into the second container 12 and to prevent it from flowing back to the first container 11, or open when the recirculation of the organic material is activated in the second container 12.

The first recirculation member 43 can be connected directly to the feed device 46 which has introduced the organic material from the first container 11 into the second container 12, or it can be connected to a stirring device 31, substantially analogous to that described with reference to fig. 3.

A second recirculation member 47 can be connected to the second container 12, configured to recirculate, in the second container 12, the gas present in it. In this way the gas which, on each occasion, is generated and stratifies in the top part of the second container 12 is constantly introduced into the solid/fluid organic material already present, to increase the bacterial load present and to increase the content of methane in the gas generated.

The second recirculation member 47, shown schematically in figs. 1 and 4, can be connected to the second container 12 in correspondence with a first recirculation aperture 48 and a second recirculation aperture 49.

The first recirculation aperture 48 can be made in a top part of the second container 12, at a height where the gas generated stratifies.

The second recirculation aperture 49 can be made in a bottom part of the second container 12, in a position where the fluid/solid organic material stratifies.

The first recirculation aperture 48 and the second recirculation aperture 49 can have respective interception and/or distribution valves to control the flow of organic material.

The second recirculation member 47 can comprise a tube 53 that fluidically connects the first recirculation aperture 48 and the second recirculation aperture 49 and a suction and compression device 52 associated with the tube 53 and which determines the transfer of the gas from the first recirculation aperture 48 to the second recirculation aperture 49.

The second recirculation member 47 can comprise a plurality of branched tubes 50 located inside the second container 12 and each provided with mixing nozzles 51 through which the gas is delivered.

A heating device 54 can be associated with the tube 53, and is configured to heat the gas passing through the tube 53.

Between the second container 12 and the third container 13 a second transfer device 55 is connected, configured to allow to transfer the organic material from the second container 12 to the third container 13.

The second transfer device 55 can be substantially analogous to the first transfer device 35 described above.

The second transfer device 55 can be connected to a pickup aperture 56 made in a bottom part of the second container 12, and an introduction aperture 57 made in a bottom part of the third container 13.

According to possible variants, the second transfer device 55 can be integrated or associated with the second recirculation member 47. In this case, suitable distribution and/or interception valves are provided, selectively activated in order to actuate the specific functions required.

A suction member 41 can also be connected to the second container 12, substantially analogous to the suction member 41 connected to the first container 11 and configured to transfer the gas generated in the second container 12 to the third container 13.

For this embodiment too, it can be provided that the suction member 41 is integrated and/or cooperates with the second transfer device 55 to allow to homogenize the solid/fluid organic material with the gas to be transferred from the second container 12.

The third container 13 can be provided with a feed device 62 connected to the second transfer device 55 and configured to introduce the solid/fluid organic material into the third container 13. The feed device 62 can be substantially analogous to the feed device 14 connected to the first container 11.

The feed device 62 can be connected in correspondence with the introduction aperture 57.

In the same way as provided for the second container 12, a first recirculation member 43 and/or a second recirculation member 47 can be connected to the third container 13, and are substantially analogous to those described above.

In particular, the second recirculation member 47 can be connected in correspondence with a discharge aperture 58, which in this case functions as the first recirculation aperture 48 as described above with reference to the second container 12, and a second recirculation aperture 49.

The discharge aperture 58 is made in a top part of the third container 13 so as to allow to suck up the gaseous fraction present therein.

The third container 13 can be provided with a discharge device 59 to discharge the liquid part of the organic material, located in correspondence with the level of the free surface.

Pickup devices 60 are connected to the third container 13, to pick up the gas inside it. The pickup devices 60 can be connected to the second recirculation member 47, for example in correspondence with the discharge aperture 58. Thanks to its energy potential, deriving from a high percentage of methane, the gaseous product can feed a combined plant for energy production.

A discharge mean 61 is also connected to the third container 13, to discharge the solid/fluid organic material contained inside it. The discharge mean 61 can be connected to the third container 13 in a bottom part thereof.

The time the organic material and/or gas generated remains in the three containers 11, 12, 13 is suitably controlled by pressure and/or temperature sensors located inside the containers 11, 12, 13, or by suitable timers.

We will now describe the functioning of the organic material.

The matrix, perfectly homogenized by the treatment devices 18, is introduced into the first container 11 in which the first digestive stage of the organic material is begun by the bacteria. To accelerate the digestive process and to obtain a better performance, the organic material is further heated during the recirculation by means of the heating device 25, and kept at a temperature of about 50-52°C.

The first phase of the methanogenesis process starts in the first container 11, substantially comparable to a UASB reactor, where a very fast digestion takes place, which maximizes the hydrolytic phase. In this phase the complex organic components of the matrix are split into simpler molecules.

The hydrolytic phase can be divided into 4 sub-phases: fibrolysis, amylolysis, proteolysis and lipolysis. Fibrolysis consists of splitting the fibers which are polysaccharides, present in the matrix, into simple sugars ((C₆H₁₀O₅)ₙ + n H₂O⇔n C₆H₁₂O₆) mainly performed by the following bacteria: Fibrobacter succinogens, Ruminococcusflavefaciens and Ruminococcus albus. Amylolysis too is the lysis of a polysaccharide into simpler sugars, but even if it is chemically similar, the splitting of the starch is performed by the following bacteria: Streptococcus bovis, Bacteroides ruminucola, Ruminobacter amylophylus, Succinimonas amylolitica, Selenomonas ruminantum, Lachnospira multiparus and Succinivibrio dextrinosolvens. Proteolysis is the splitting of the proteins into their individual amino acids, and is performed by the following bacteria: Prevotella ruminicola, Butyrivibrio fibrisolvens, Streptococcus bovis and Ruminobacter amylophylus.

Lipolysis is the splitting of the mono-, di- and tri- glycerides into fatty acids and glycerol (CH₂OCOR¹-CHOCOR²-CH₂OCOR³+ 3 H₂O⇔CH₂OH-CHOH-CH₂OH + R¹COOH + R²COOH + R³COOH). In this phase the first VFA (Volatile Fatty Acids) are formed, in which acetic acid has an important role in the methanogenesis process. The bacteria that perform these reactions are essentially: Butyrivibrio fibrisolvens, Treponema bryantii, Eubacterium spp., Ruminococcus albus, Fusocilus spp., Micrococcus spp.

At the end of the hydrolytic phase, and always inside the first container 11, the simpler molecules are ready to be attacked by other bacteria which perform the second phase of the methanogenesis process, said acidogenesis phase.

The hydrolytic phase and the acidogenic phase occur inside the first digestive phase, that is, in the first container 11, and have a duration (or retention time) varying between 6 and 16 hours. During this period it is possible to command the recirculation of at least part of the organic material in the first container 11. During recirculation, it can be provided to heat the organic material. In this phase the simple sugars are transformed into two molecules of propionic acid, while the amino acids are subjected to a process of deamination. In this way, the organic matrix becomes more acid and is ready for the second digestive stage, the acetogenic phase which consists in transforming the volatile fatty acids into acetic acid.

From the chemical point of view, the acetogenesis process can be schematized by these reactions: (CH₃CH₂CH₂COO⁻ + 4 H₂O⇔CH₃COO⁻ + H⁺ + HCO³⁻ + 3 H₂), (CH₃CH₂COO⁻ + 3 H₂O ⇔CH₃COO⁻ + H⁺ + HCO³⁻ + 3 H₂) and (CH₃CH₂OH + 2 H₂O⇔CH₃COO⁻ + 2 H₂ +H⁺), in which the volatile fatty acids like butyric and propionic are transformed into acetic acid.

This process takes the name of Obligate Hydrogen Producing Acetogens (O.H.P.A.) and is performed by the following bacteria: Desulfotomaculum termocisternum, Desulfotomaculum Termobenzoicum termosintrophicum, Pelotomaculum termopropionicum, Methanotermobacter spp., Methanosarcina spp.

At the end of the second phase (acetogenesis), when by now in the organic matrix there is mainly acetic acid and a large quantity of bacteria, including the methanogenic bacteria, the whole content is pumped into the second container 12 and then into the third container 13, where the last stage of the process is maximized, the stage where the most important digestive process takes place: the methanogenic phase.

In particular, the organic material is maintained for about 5-7 days in the second container 12 and for another 5-7 days in the third container 13. During this period, the solid/fluid part of the organic material is constantly recirculated by the first recirculation member 43 while the gaseous fraction is recirculated by the second recirculation member 47.

The organic matrix present in the third container 13 is rich in carboxyl acid (acetic acid) which during the process is transformed into methane through the various bacterial pathways: these chemical transformations can all be traced to three main pathways: the acetotrophic, methylotrophic and hydrogenotrophic pathways, which reactions take place simultaneously in the third stage.

The acetotrophic pathway is the bacterial pathway with the simplest chemism: CH₃COOH ⇔CH₄ + CO₂ and is performed by Methanosarcina acetivorans, Methanosaeta concilii and Methanosaeta spp.

The methylotrophic and hydrogenotrophic pathways require the acetic acid to be transformed into other chemical compounds (CH₃COOH + R-H⇔CH₃-R + H₂ + CO₂) performed by Acetobacterium woodii and Clostridium aceticum. The products of this reaction are fundamental for the chemism of both bacterial pathways.

The methylotrophic pathway provides to form methane through the following reaction: (CH₃-R+ H₂⇔CH₄ + R-H) thanks to Methanosarcina Barkeri, Methanococcoides spp., Methanohalobium spp., Methanohaliphylus spp., Methanolobus spp. This pathway is called methylotrophic because it is the methyl groups that react with the molecules of hydrogen to form the methane.

The hydrogenotrophic pathway produces methane by the following reaction: (CO₂ + 4H₂ ⇔ CH₄ + 2 H₂O) performed by the bacteria: Methanobacterium jormicium, Methanobrevibacter arboriphylus, Methanospyrillum hungatei, Methanoculleus bourgernsis and Methanosphaera spp. The hydrogenotrophic pathway takes its name precisely from the molecules of hydrogen needed to react with the carbon dioxide to form the methane.

At the end of the whole continuous multiphase anaerobic digestion process, we obtain a biogas that is very rich in methane (70% volume) with percentages of carbon dioxide, hydrogen, oxygen, hydrogen sulfide and other gases below the standard processes, the digestate is treated by a solid/liquid separator, the very watery liquid part is recirculated in the digestion process while the solid part can be used as fertilizer.

The embodiments described here allow to maximize the production of methane, as a sub-product of the gaseous product, and simultaneously allow to reduce the overall size of a plant for the production of gas. Therefore, this embodiment allows to install a plant for the production of gas even in small zootechnical entities, because it entails low initial investment costs and low management costs.

Merely by way of example, Applicant has verified that for a farm with fifty head of lactating cattle, the potential production can be up to 20k W/h.

It is clear that modifications and/or additions of parts may be made to the plant for the production of gas as described heretofore, without departing from the field and scope of the present invention.

It is also clear that, although the present invention has been described with reference to some specific examples, a person of skill in the art shall certainly be able to achieve many other equivalent forms of plant for the production of gas, having the characteristics as set forth in the claims and hence all coming within the field of protection defined thereby.

## Claims

1. Plant for the production of gas, by means of digestion of an organic material, comprising a first container (11) configured to contain said organic material and in which at least part of the digestive process occurs, **characterized in that** it comprises at least a second container (12) and a first transfer device (35) connected between the first container (11) and the second container (12) and configured to transfer said organic material from said first container (11) to said second container (12), and **in that** a respective first recirculation member (24, 43), configured to generate a recirculation of said organic material in the respective container, is connected to at least one of either said first container (11) or said second container (12).

2. Plant as in claim 1, **characterized in that** said first recirculation member (24, 43) is connected to said first container (11) and to said second container (12).

3. Plant as in claim 1 or 2, **characterized in that** it comprises a third container (13) and a second transfer device (55) connected between the second container (12) and the third container (13) and configured to transfer said organic material from said second container (12) to said third container (13).

4. Plant as in claim 3, **characterized in that** a respective first recirculation member (43) is connected to said third container (13).

5. Plant as in claim 3 or 4, **characterized in that** the first container (11), the second container (12) and the third container (13) are installed one above the other.

6. Plant as in any claim hereinbefore, **characterized in that** said first recirculation member (24, 43) is connected to a stirring device (31) located in the first container (11) and/or in the second container (12) and configured to introduce the recirculated organic material with a desired degree of turbulence and stirring.

7. Plant as in any claim hereinbefore, **characterized in that** a second recirculation member (47) is connected to at least said second container (12), and is configured to carry out a recirculation of the gas present in the second container (12).

8. Plant as in any claim hereinbefore, **characterized in that** it comprises heating devices (25, 40) configured to heat said organic material.

9. Plant as in any claim hereinbefore, **characterized in that** it comprises a feed device (14) connected to said first container (11) and configured to introduce said organic material into the latter, and **in that** devices to treat the organic material which is introduced therein are associated to said feed device (14).

10. Method for the production of gas by means of digestion of an organic material, which provides to introduce and temporarily keep said organic material in a first container (11) in order to start the digestive process, **characterized in that** it provides at least to transfer said organic material from said first container (11) into a second container (12), and to keep said organic material in said second container (12), and **in that** in at least one of either the first container (11) or said second container (12) a recirculation of the material contained therein is generated.
